(19) Europäisches Patentamt

European Patent Office

Office européen des brevets

(11) **EP 1 052 290 A2**

(12) **EUROPÄISCHE PATENTANMELDUNG**

(43) Veröffentlichungstag:
**15.11.2000 Patentblatt 2000/46**

(51) Int. Cl.[7]: **C12P 17/10**, C09B 69/10,
A61K 7/42, C08G 61/12

(21) Anmeldenummer: **00109282.4**

(22) Anmeldetag: **28.04.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **12.05.1999 DE 19921710**

(71) Anmelder: **Clariant GmbH**
**65929 Frankfurt am Main (DE)**

(72) Erfinder:
- **Turowski-Wanke, Angelika, Dr.**
  **65779 Kelkheim (DE)**
- **Berghoff, Kornelia, Dr.**
  **14473 Potsdam (DE)**

(54) **Biomelanin**

(57)   Biomelanin, hergestellt durch eine Fermentation des Pilzes Podospora anserina der Familie Sondariaceae in einem geeigneten Nährmedium, enthaltend mindestens eine Kohlenstoff-, Phosphat- und Eiweißquelle bei einem pH von 5 - 7 und anschließender Aufarbeitung sowie kosmetische Zubereitungen, die dieses Biomelanin enthalten.

**EP 1 052 290 A2**

Printed by Xerox (UK) Business Services
2.16.7 (HRS)/3.6

**Beschreibung**

Der Begriff Melanin, abgeleitet von den griechischen Wörtern mel (schwarz) und anin (Amin), umfasst ein breite Gruppe von natürlichen und synthetischen Pigmente, bestehend aus heterogenen Polymeren mit unterschiedlichen Monomereinheiten mit Indol-, Pyrrol-, Phenol- und Quinon Strukturelementen. Natürlich vorkommende Melanine sind die durch enzymatische Oxidation der Aminosäure Tyrosin entstehenden gelblich bis braunen (Phäo-Melanin) oder dunkelbraun bis schwarzen (EU-Melanin) Pigmente bei Tieren und Menschen, die von den Melanocyten in der Epidermis oder in einer darunterliegenden Zellschicht gebildet und abgelagert werden. Melanine bewirken die Färbung der Haut und ihrer Anhangorgane (Haare, Federn) sowie der Regenbogenhaut der Augen. Melanine befinden sich darüberhinaus im Herzen, in der Leber, in den Arterien, in der Epiphyse, den Testikeln und den Ovarien. Entsprechend der chemischen Struktur der Grundbausteine werden Melanine in unlösliche, schwarze Eumelanine auf Basis von Poly-5,6-Indolquinonen und die im alkalischen Medium löslichen rötlich braunen Phäomelanine auf Basis von Polydihydrobenzothiazinen eingeteilt.

Melaninähnliche Pigmente kommen in nahezu allen Lebensformen, wie Pflanzen, Pilzen und Bakterien vor. Anders als tierische und menschliche Melanine, die intrazellulär aus Tyrosin entstehen, werden pilzliche Pigmente, auch Allomelanin genannt, meist extrazellulär durch enzymatische Oxidation von Phenolderivaten, die nicht zwingend Stickstoff enthalten müssen, beispielsweise Catechol, Quinon und deren Derivate gebildet.

Natürliche Melanine werden vorwiegend durch Extraktion des Sepia-Melanins des Tintenfisches gewonnen. Synthetisch erzeugte Melanine werden durch enzymatische oder chemische Oxidation (EP 4416890, WO 94/25531, US 5006331) von Melaninvorstufen, beispielsweise Indolderivate, insbesondere 5,6-Dihydroxyindol, Indolin, D- und L-Dihydroxyphenylalanin (DOPA), Tyramine oder Tyrosin erhalten. Weiterhin ist bekannt, daß synthetisches Melanin mit Hilfe von Pflanzenzellen (Mohn) aus den oben genannten Vorstufen hergestellt werden kann (WO 94/12653).

In US 5814495 und WO 9412644 wird die biotechnologische Produktion von Melanin durch extrazelluläre Tyrosinase von Tyrosin induziert durch bakterielle Mikroorganismen (Streptomyces) beschrieben.

Melanine können aufgrund ihrer chemischen Struktur Energie wie beispielsweise Sonnenenergie, Radar, Röntgenstrahlung, aber auch hörbare Frequenzen (Musik) absorbieren, weiterleiten oder auch speichern. Des weiteren sind Melanine gute Radikalfänger und Antioxidantien und schützen Haut und Gewebe vor Schädigungen durch freie Radikale und Peroxide.

Aufgrund dieser Eigenschaften finden synthetische und natürliche Melanine breite Anwendungen, insbesondere in der Kosmetik. Zahlreiche Patent- und Anmeldeschriften (DE 4221269, EP 709423, WO 94/25531, WO 9412653, EP 518773) beschreiben melaninhaltige Hautpflegemittel, Sonnenschutzcremes, Makeup, Antiaging Kosmetik etc. zum Schutz von Haut und Haaren vor Schädigungen durch UV-Strahlung.

Die industrielle Produktion von Melaninen erfordert aufwendige und kostenintensive Technologien. Des weiteren sind Melanine nicht oder nur mäßig löslich in organischen und anorganischen Lösungsmitteln, insbesondere schlecht löslich oder unlöslich in Wasser oder können erst in Gegenwart von Additiven, beispielsweise durch Zugabe von Trypsin oder Triethanolamin in Wasser gelöst werden. Melaninähnliche Pigmente können synthetisch durch oxidative Polymerisation aromatischer Monomere, beispielsweise p-Aminobenzolsäure, 3-Aminotyrosin, 5,6-Dihydroxyindol (WO 96/25920) erzeugt werden. Die so erhaltenen farbigen Polymere sind teilweise wasserlöslich, ihre biologische Aktivität, insbesondere bezüglich ihrer Radikalfängereigenschaften sind nicht bekannt. Es bestand daher die Aufgabe, ein biologisch aktives Melanin mit guter Schutzwirkung gegen den Einfluß der UV-Strahlung auf den Organismus, bereitzustellen, das toxikologisch unbedenklich, wasserlöslich und auch im industriellen Maßstab herstellbar ist.

Es wurde gefunden, daß der Pilz Podospora anserina der Familie Sordariaceae und auch ihre Mutanten in der Lage sind, "in vivo" intrazellulär wasserlösliche Melanine, die aus stickstoffhaltigen organischen Gruppen aufgebaut sind, zu bilden. Überraschenderweise ist es möglich, im Gegensatz zu den Melaninen gemäß dem Stand der Technik die Melaninbildung zu jedem Zeitpunkt zu stoppen und damit den Polymerisationsgrad, die Wasserlöslichkeit, die Farbe, Radikalfängereigenschaft und weitere Wirkungen der erfindungsgemäßen Melanine in einem bestimmten Bereich zu steuern. Das erfindungsgemäße Biomelanin zeigt alle für Melanine typischen Eigenschaften und kann insbesondere für kosmetische Anwendungen natürliche und synthetisch erzeugte Melanine oder melaninähnliche Pigmente ersetzen.

Gegenstand der Erfindung sind

Biomelanine, hergestellt durch eine Fermentation des Pilzes Podospora anserina der Familie Sordariaceae in einem geeigneten Nährmedium, enthaltend mindestens eine Kohlenstoff-, Phosphat- und Eiweißquelle bei einem pH von 5 -7 und anschließender Aufarbeitung sowie kosmetische Zubereitungen, die diese Biomelanine enthalten. Die fermentative Bildung der Biomelanine in den Zellen des Pilzes Podospora anserina kann beispielsweise wie folgt durchgeführt werden.

Zur Gewinnung der Vorkultur wird der gefrierkonservierte Pilz (Impföse oder ein Agarstückchen), auf eine Agarplatte mit Biomalzmedium gegeben. Der sich im Laufe von 7 bis 14 Tagen tiefbraun verfärbende Pilz wird von den Agarplatten abgehoben und unter sterilen Bedingungen mit Hilfe von Glasperlen in Saline zerkleinert. Die so gewonnene Suspension wird in ein geeignetes steriles Medium überführt, das beispielsweise aus Cornsteep, Malzextrakt, einer Phosphatquelle, beispielsweise Kaliumdihydrogenphosphat und Zucker (Glucose, Saccharose) pH 5 - 7, insbesondere
pH 6 - 6,8, bevorzugt pH 6,5 (eingestellt mit Alkalihydroxid) besteht. Die Gewichtsmengen der Mediumzusätze (in g/l) Cornsteep / Malzextrakt 1 $KH_2PO_4$ /Glucose / Saccharose können 7,5 /2,0/0,5/100,0 /5,0 sein. Die Anzucht erfolgt als Schüttelkultur
(50 bis 200 rpm, bevorzugt 100 bis 150 rpm, besonders bevorzugt 120 bis 130 rpm) bei 15 °C bis 50 °C, bevorzugt bei 25 °C bis 35 °C, besonders bevorzugt bei 30 °C unter aeroben Bedingungen innerhalb von 3 bis 6 Tagen, bevorzugt 3 bis 5 Tagen, besonders bevorzugt 4 Tagen.

Der Start der Fermentation erfolgt mit steriler Überführung dieser Vorkultur in die Passagenkultur. Dabei sind 0,5 bis 2 %, bevorzugt 1 bis 2 % Inokulumsmenge anzusetzen. Als Mediumszusätze dienen beispielsweise Cornsteep, Malzextrakt, $KH_2PO_4$, Glucose und Pharma Media in den Gewichtsmengen 7,5/2,0/0,5/100,0/5,0 g/l bei einem pH wie zuvor angegeben (eingestellt mit Alkalihydroxid). Dem Mediumzusatz können Antischaummittel, beispielsweise Siliconentschäumer zugesetzt werden. Um Bräunungsreaktionen während der Sterilisation zu minimieren, können der Zucker und die anderen Mediumsbestandteile getrennt autoklaviert werden. Dazu wird das zuckerfreie Medium im Fermenter mit etwa 90 % des Wassers sterilisiert und der Zucker mit etwa 10 % des Wassers extern behandelt. Bei der Sterilisation des Mediums sollten ca. 100 ml Antischaummittel je 1000 l Fermentationsmedium vorgelegt werden. Die Fermentationszeit beträgt 4 bis 10 Tage, bevorzugt 5 bis 6 Tage, besonders bevorzugt 5,7 Tage bei 15 °C bis 50 °C, bevorzugt bei 20 °C bis 30 °C, besonders bevorzugt bei 25 °C. Die Belüftungsrate beträgt 0,6 bis 1 vvm (in großen Fermentern 0,6 bis 0,9 vvm), der Anteil des Gelöstsauerstoffes sollte mindestens 60 % betragen. Die Schaumdämpfung sollte möglichst gering gehalten werden (< 0,5 ml/l Medium).
Zur Qualitätskontrolle erfolgt die Prüfung der mikrobiologischen Reinheit am 4. Tag (Ausstrich: Maismedium + 1,2 % Agar, Caso-Agar).
Nach Abschluß der Fermentation werden die braunen bis schwarzbraunen Pellets als Inokulum für die nächste Passage oder den Produktionsfermenter eingesetzt. In Abhängigkeit von der Größe des Produktionsfermenters kann diese Passagierung einmal oder mehrmals vorgenommen werden.

Für den Produktionsfermenter gelten grundsätzlich die gleichen Bedingungen wie bei der Passagierung. Als Mediumszusätze (in g/l) dient beispielsweise Cornsteep, Malzextrakt, $KH_2PO_4$, Glucose und Pharma Media in den Gewichtsmengen 7,5/ 2,0 / 0,5 / 100,0 / 5,0 bei einem pH wie oben angegeben (eingestellt mit Alkalihydroxid). Zur Vermeidung starker Schaumbildung werden 100 ml eines der oben genannten Antischaummittel je 1000 l Fermentationsmedium vorgelegt. Die Beimpfung (0,5 bis 2 %, bevorzugt 1 bis 2 %) der Produktionsfermentation erfolgt direkt mit der Vorkultur oder Passage. Die Qualitätskontrolle wird am 4. Tag durch Prüfung der mikrobiologischen Reinheit wie oben beschrieben durchgeführt. Die Fermentationstemperatur beträgt
15 - 50 °C, bevorzugt 20- 30 °C, insbesondere 25 °C.

Die Fermentation kann zu jedem Zeitpunkt abgestoppt werden. Bei einer Fermentationszeit von 4 Tagen werden hellbraune Biomelanine gewonnen, nach 8 bis 10 Tagen Fermentation unter oben genannten Bedingungen entstehen dunklere Melanintypen. Bevorzugt sind daher Fermentationszeiten von 4 bis 8 Tagen.

Ergebnis der Fermentation ist zunächst die Biomasse mit dem enthaltenen Biomelanin. Zur Gewinnung diesen Biomelanins wird die Biomasse aus der Kulturlösung abgetrennt, gewaschen und aufgeschlossen. Mit einer Pufferlösung erfolgt die Extraktion des Produktes und nach Einengen der Lösung wird das Produkt lyophilisiert.

Die Abtrennung der Biomasse (braune bis schwarzbraune Pellets) erfolgt durch Zentrifugation oder Filtration. Die Kulturlösung wird verworfen. Die Zellmasse wird mit 10 -20 % Trinkwasser bezogen auf das Fermentationsmedium nachgespült und von restlichen Mediumsbestandteilen berfreit. Die Biomasse kann in Portionen eingefroren und bei -20 °C gelagert werden.

Der notwendige Pufferbedarf wird durch Bestimmung der Biomassentrockensubstanz (BMTS) und des Wassergehaltes ermittelt. Dabei ist die Feuchtbiomasse (FBM) gleich der Auswaage der gesamten Produktmenge; der Wassergehalt wird über die Trocknung der Feuchtbiomasse bestimmt. Die Biomassetrockensubstanz (BMTS) stellt das Produkt aus FBM (g) und Wassergehalt dar. Die Puffermenge je Extraktionsschritt berechnet sich nach der Formel:

$$\text{Puffermenge je Extraktionsschritt [ml] = BMTS (g) * 21,1 (Bedarfsfaktor)}$$

Zur Extraktion wird ein Phosphatpuffer nach Sörensen oder andere gängige Puffersysteme, beispielsweise Citratpuffer verwendet.

Nach Mischung der Biomasse mit der halben Puffermenge der ersten Extraktion erfolgt der Aufschluß der Zellen mittels eines Hochdruckhomogenisators oder in einer Koloidmühle. Im kleineren Maßstab kann auch ein Rührkessel mit Standardturbinenrührern und einer Umfangsgeschwindigkeit von 7 m/s eingesetzt werden (Aufschlußdauer: 15 min). Bei Einsatz eines alternativen Verfahrens ist die Qualität des Zellaufschlusses mit den vorgeschlagenen Verfahren zu vergleichen.

In der ersten Extraktionsstufe wird der Zellaufschluß mit der zweiten Hälfte der Puffermenge aufgestockt und die erste Extraktion unter leichtem Rühren für 30 min durchgeführt. Danach erfolgt die Abtrennung der Zellbestandteile vom Extrakt und die Pufferextrakte werden gesammelt.

Die Biomasse wird in der zweiten und dritten Extraktion mit jeweils der berechneten Puffermenge analog zur ersten Extraktion (30 min unter leichtem Rühren) versetzt. Die Zellmasse wird anschließend verworfen. Die Extrakte sind möglichst zeitnah zu verarbeiten. Sie können maximal für 24 Stunden bei +4 °C gelagert werden oder in konservierungsmittelhaltigen Lösungen überführt werden. Als Konservierungsmittel geeignet sind beispielsweise Phenoxyethanol, Fomaldehydlösung, Parabene, Pentandiol oder Sorbinsäure.

Die vereinigten Extrakte werden in einem Vakuumverdampfer bei 50 bis 60 °C weitgehend entwässert. Dabei kann im günstigsten Fall eine Einengung auf 10 - 15 % erfolgen. Bei zu starker Einengung (Schlammbildung) kann durch Zugabe von etwas Wasser eine pumpfähige Suspension erzeugt werden.

Die eingeengten Extrakte werden zur schonenden Konservierung mittels Gefriertrocknung auf eine Restfeuchte < 5 % (vorzugsweise 2 - 3 %) getrocknet. Der Vorgang dauert pro Charge ca. 24 bis 48 Stunden.

Die Produktqualtität ist wie folgt definiert:

Spezifikation:

| Keimgehalt | < 100 KBE/g |
|---|---|
| Korngröße | 50 - 500 µm |
| Farbe | goldbraunes Pulver |
| pH-Wert (0,5 %ige wäßrige Lösung) | 5,0 - 6,0 |

Zur Einstellung der goldbraunen Farbe gemäß Spezifikation ist es ggf. notwendig, eine Nacherhitzung des Produktes vorzunehmen. Das Produkt wird hierzu in geringer Schichtdicke (< 2 cm) auf Blechen (Trennmittel) auf 105 °C für 15 bis 90 min, vorzugsweise 30 bis 45 min, erhitzt. Grundsätzlich ist mit einer geringen Menge ein Vortest durchzuführen.

Das farbeingestellte Endprodukt wird bei < 25 °C in einer Mühle zu Pulver vermahlen (Korngröße gemäß Spezifikation) und das Produktpulver anschließend feuchtigkeitsunempfindlich (Folie) in einem festen Gebinde verpackt.

Die erfindungsgemäß in den Zellen des Pilzes Podospora anserina und/oder dessen Mutanten fermentativ gewonnenen Biomelanine haben in Abhängigkeit der Fermentationsdauer unterschiedliche Bräunungsstufen von hellbraun bis braun und einen unterschiedlichen Polymerisationsgrad. Die bei Fermentationszeiten von 4 bis 8 Tagen gewonnenen hellbraunen bis braunen Biomelanine sind zu mehr als 95 % wasserlöslich.

Die Molekulargewichte der erfindungsgemäßen Biomelanine mit den Bräunungsstufen von hellbraun bis braun liegen deutlich unter 5 000 Dalton. Die Molekulargewichtsbestimmung erfolgte mittels HPLC. Für andere enzymatisch erzeugte Melanine (WO 98/ 34606) wurden Molekülgewichte von mindestens 10 000 Dalton gemessen oder können wegen der Unlöslichkeit der Melanine nicht bestimmt werden. Die durch chemische Oxidation aus den Monomeren Tyrosin oder Dihydroxyphenylalanin oder Dihydroxyindol-2-Carbonsäure oder 3-Aminotyrosin erzeugten Melanin-Polymere haben, wie in WO 96/25920 beschrieben, Molekulargewichte von mehr als 20 000 Dalton.

Die erfindungsgemäßen Biomelanine zeigen im UV-Spektralbereich zwischen 190 nm und 900 nm zwei Absorptionsmaxima bei 215 nm und 260 nm, wobei die Extinktionen mit zunehmendem Bräunungsgrad des Melanins zunehmen.

Die UV-Spektren der Melanine wurden von 0,1 %igen wäßrigen Lösungen aufgenommen.

Die erfindungsgemäßen Biomelanine weisen Elektronenspinresonanz-Signale moderater Intensität (Intensität ESR / mg: 16) auf, deren Breite (Delta Hpp = 10 G, 12 G) und g-Wert (g = 2,004) mit Literaturwerten für Melanine übereinstimmt. Das ESR-Spektrum des erfindungsgemäßen pulvrigen Biomelanins wurde bei Raumtemperatur als Pulver aufgenommen. Das ESR-Signal von Sepia-Melanin wird in der Literatur mit einem g-Wert von 2,00405, das natürliche Melanin aus dem Haar eines Asiaten mit g = 2,00384, synthetisches Melanin aus 5,6-Dihydroxyindol mit g = 2,00376 angegeben.

Melaninen werden für den Menschen lebens- und überlebenswichtige Schutzfunktionen vor UV-Strahlung zugeschrieben. Als Folge der UV-Bestrahlung bilden sich in der Haut verschiedene aktive Sauerstoffverbindungen. Diese spielen eine wichtige Rolle als auslösende oder verstärkende Faktoren für zahlreiche biochemische Prozeße. Hierzu gehören u. a. Erythembildung, Immunsuppression, Hautalterung (Faltenbildung und Verlust an Elastizität) und wahrscheinlich auch die Enstehung von Hautkrebs. Durch UV-Licht erzeugte reaktive Sauerstoffspezies und freie Radikale führen zur Lipid-Peroxidation von Biomembranen. Eine Schutzfunktion von Melanin wird auf dessen Radikalfängereigenschaft und antioxidative Wirkung zurückgeführt. Melanine können die durch UV-Strahlung erzeugten Sauerstoffradikale in für den Organismus unschädliche Sauerstoffverbindungen bzw. Sauerstoff überführen oder die Oxidation reaktiver Moleküle durch Sauerstoff inhibieren.

Der Mechanismus einer durch freie Radikale initiierten Lipidperoxidation ist eine Kettenreaktion. D. h. ein einziges freies Radikal kann viele Lipidperoxide bilden. Um die Kettenreaktion zu stoppen, muß das freie Radikal mit einem zweiten Radikal reagieren. Melanine sind stabile Radikale und können als Radikalfänger und Antioxidantien die Bildung von Lipidperoxiden verhindern oder reduzieren.

Die erfindungsgemäßen Biomelanine können, wie Untersuchungen an Leberzellen der Maus und der Ratte zeigen, die Lipidperoxidation inhibieren.

Im Testversuch werden 1,5 mg Maus- bzw. Rattenlebermikrosome mit 30 Mikrogramm Melanin bei 37 °C 20 Minuten erwärmt. Anschließend wird durch Zugabe von 100 Mikroliter Ascorbinsäure und 100 Mikroliter $FeCl_3$ die Lipidperoxidation initiiert. Diese Reaktion wird nach 1 Stunde bei 37 °C durch Zugabe einer 20 %igen Trichloressigsäure und 1 %iger 2-Thiobarbitursäure bei 90 °C innerhalb von 20 Minuten gestoppt. Der Anteil von Lipidperoxid wird photospektrometrisch (535 nm) bestimmt. Das Ergebnis wird in nmol Malondialdehyd (MDA)/mg Protein/1 h angegeben. Zum Vergleich der in Gegenwart des erfindungsgemäßen Biomelanins gefundenen Werte (1) werden die Lipidperoxidkonzentrationen in Abwesenheit eines Initiators (2) sowie die Lipidperoxidation in Gegenwart eines Initiators ohne Melaninzugabe (3) sowie die Lipidperoxidationkonzentration in Gegenwart eines Melanins, das extrazelluläre Tyrosinase von Tyrosin induziert das durch bakterielle Mikroorganismen (Streptomyces), wie in WO 94/12644 beschrieben, erzeugt wird (4) nach gleichem Verfahren ermittelt.

Tabelle

| Lipidperoxidation in Mauslebermikrosomen | |
|---|---|
| Probe | nmol MDA/mg Protein/h |
| 1 | 1,20 |
| 2 | 0,85 |
| 3 | 1,80 |
| 4 | 1,80 |

Das erfindungsgemäße Biomelanin kann in unterschiedliche kosmetische Produkttypen, beispielsweise Lotionen, Cremes, Balm, Badeöle, Gele, Sprays, Schäumen, Puder, Aerosolen, Sticks, Pasten, Salben, Make-up entweder auf Basis einer Lösung, auf der Basis einer Emulsion oder auch in verkapselter Form eingearbeitet werden.

Als Lösung formulierte kosmetische Produkte erfordern physiologisch gut verträgliche Lösungsmittel. Sie enthalten überwiegend Wasser als Lösungsmittel der wäßrige Puffersysteme, beispielsweise Triethanolamin und Phosphate. Emulsionen, enthaltend das erfindungsgemäße Melanin, schließen Öl-in-Wasser-, Wasser-in-Öl- und auch Öl-in-Wasser-in-Silicon-Emulsionstypen ein.

Die erfindungsgemäßen Melanine können nach gängigen Methoden, beispielsweise mit Phospholipiden oder Cyclodextrinen, verkapselt werden und in dieser Form länger am Wirkungsort verbleiben.

Das erfindungsgemäße Melanin kann in den kosmetischen Zubereitungen, beispielsweise in Sonnenschutzmitteln, Pflegemitteln für Haut und Haare, Make-up, Antiaging-Kosmetik in Mengen von 0,001 Gew.-% bis 10 Gew.-%, bevorzugt 0,005 bis 5 Gew.-% eingesetzt werden.

Die erfindungsgemäßen Mittel können als weitere Hilfs- und Zusatzstoffe Lichtschutzstoffe (UV-Filter, Pigmente, Mikropigmente), Antioxidantien, Tenside, Emulgatoren, Überfettungsmittel, Trägeröle, Fette, Wachse, Stabilisatoren, biogene Wirkstoffe, Glycerin, Konservierungsmittel, Perlglanzmittel, Verdicker, organische Lösungsmittel, Silikone, Weichmacher, Hydrationsmittel, Füllstoffe, Sequestriermittel, Treibmittel, Farb- und Duftstoffe enthalten.

Als UV-Filter kommen in Betracht 4-Aminobenzoesäure, 3-(4'-Trimethylammonium) benzyliden-bornan-2-onmethylsulfat, 3,3,5-Trimethyl-cyclohexylsalicylat, 2-Hydroxy-4-methoxybenzophenon, 2-Phenylbenzimidazol-5-sulfonsäure und ihre Kalium-, Natrium- und Triethanolaminsalze, 3,3'-(1,4-Phenylendimethin)-bis-(7,7-dimethyl-2-oxobicyclo[2.2.1]-heptan-1-methansulfonsäure und ihre Salze, 1-(4-tert.-Butylphenyl)-3-(4-methoxyphenyl)propan-1,3-dion, 3-(4'-Sulfo)-benzyliden-bornan-2-on und seine Salze, 2-Cyan-3,3-diphenyl-acrylsäure-(2-ethylhexylester), Polymer von N-[2(und 4)-(2-oxoborn-3-ylidenmethyl)benzyl]-acrylamid, 4-Methoxy-zimtsäure-2-ethyl-hexylester, ethoxyliertes Ethyl-4-amino-benzoat, 4-Methoxy-zimtsäureisoamylester, 2,4,6-Tris[p-(2-ethylhexyloxycarbonyl)anilino]-1,3,5-triazin, 2-(2H-benzotriazol-2-yl)-4-methyl-6-(2-methyl-3-(1,3,3,3-tetramethyl-1-(trimethylsilyloxy)-disiloxanyl)propyl)phenol, 4,4'-[(6-[4((1,1-dimethylethyl)-amino-carbonyl) phenylamino]-1,3,5-triazin-2,4-yl)diimino]bis-(benzoesäure-2-ethylhexylester), 3-(4'-Methylbenzyliden)-D,L-campher, 3-Benzyliden-campher, Salicylsäure-2-ethylhexylester, 4-Dimethylaminobenzoesäure-2-ethylhexylester, Hydroxy-4-methoxy-benzophenon-5-sulfonsäure (Sulisobenzonum) und das Natriumsalz, 4-Isopropylbenzylsalicylat und Mischungen daraus.

Als Pigmente/Mikropigmente werden mikrofeines Titandioxid und Zinkoxid eingesetzt. Als Antioxidantien eignen sich Superoxid-Dismutase, Tocopherol (Vitamin E) und Ascorbinsäure (Vitamin C).

An nichtionischen Tensiden kommen in Betracht: Alkyl- und/oder Alkenyloligoglycoside, Fettalkoholpolyglycolether, Fettsäurepolyglycolester, Fettsäureamidpolyglycolether, Fettaminpolyglycolether, alkoxylierte Triglyceride, Fettsäureglucamide, Polyölfettsäureester, Zuckerester, Sorbitanester und Polysorbate und/oder alkoxylierte Fettalkohole.

Bevorzugte anionische Tenside sind $C_8$-$C_{20}$-Fettsäure-alpha-methylestersulfonate, Alkylethersulfate und sek. Alkansulfonate.

Bei den sekundären Alkansulfonaten kann die Alkylgruppe entweder gesättigt oder ungesättigt, verzweigt oder linear und gegebenenfalls mit einer Hydroxylgruppe substituiert sein. Die Sulfogruppe ist statistisch über die gesamte C-Kette verteilt, wobei die primären Methylgruppen am Kettenanfang und Kettenende keine Sulfonatgruppen besitzen. Die bevorzugten sekundären Alkansulfonate enthalten lineare Alkylketten mit 9 bis 25 Kohlenstoffatomen, bevorzugt von 10 bis 20 Kohlenstoffatome und besonders bevorzugt 13 bis 17 Kohlenstoffatome. Das Kation ist Natrium, Kalium, Ammonium, Mono-, Di- oder Triethanolammonium, Calcium oder Magnesium und Mischungen davon. Natrium als Kation ist der Einfachheit halber bevorzugt.

Die anionischen Tenside, die für die vorliegende Erfindung geeignet sind, weisen Tensideigenschaften auf und sind wasserlöslich oder in Wasser dispergierbar.

Alkylsulfate sind hier wasserlösliche Salze oder Säuren der Formel $ROSO_3M$, worin R bevorzugt ein $C_{10}$-$C_{24}$-Kohlenwasserstoffrest, bevorzugt ein Alkyl- oder Hydroxyalkylrest mit $C_{10}$-$C_{20}$-Alkylkomponenten, besonders bevorzugt ein $C_{12}$-$C_{18}$-Alkyl- oder Hydroxyalkylrest darstellt ist. M ist Wasserstoff oder ein Kation, z. B. Natrium, Kalium, Lithium oder Ammonium oder substituiertes Ammonium, z. B. Methyl-, Dimethyl- und Trimethylammoniumkationen und quaternäre Ammoniumkationen, wie Tetramethylammonium- und Dimethylpiperidiniumkationen und quartäre Ammoniumkationen, abgeleitet von Alkylaminen, wie Ethylamin, Diethylamin, Triethylamin und Mischungen davon.

Ein weiteres geeignetes anionisches Tensid ist Alkylbenzolsulfonat. Die Alkylgruppe kann entweder gesättigt oder ungesättigt, verzweigt oder linear und gegebenenfalls mit einer Hydroxylgruppe substituiert sein. Die bevorzugten Alkylbenzolsulfonate enthalten lineare Alkylketten mit 9 bis 25 Kohlenstoffatomen, bevorzugt von 10 bis 13 Kohlenstoffatomen, das Kation ist Natrium, Kalium, Ammonium, Mono-, Di- oder Triethanolammonium, Calcium oder Magnesium und Mischungen davon.

Weitere geeignete anionische Tenside sind Carboxylate, z. B. Fettsäureseifen und vergleichbare Teside. Die Seifen können gesättigt oder ungesättigt sein und können verschiedene Substituenten, wie Hydroxylgruppen oder Alpha-Sulfonatgruppen enthalten. Bevorzugt sind lineare gesättigte oder ungesättigte Kohlenwasserstoffreste als hydrophobe Komponente in den Seifen. Üblicherweise enthalten die hydrophoben Komponenten 6 bis 30 Kohlenstoffatome, bevorzugt 10 bis 18 Kohlenstoffatome.

Weitere anionische Tenside sind Salze von Acylaminocarbonsäuren, die durch Umsetzung von Fettsäurechloriden mit Natriumsarkosinat im alkalischen Medium entstehen (Acylsarcosinate) sowie Fettsäure-Eiweiß-Kondensationsprodukte, die durch Umsetzung von Fettsäurechloriden mit Oligopeptiden erhalten werden. Tensidcharakter haben auch die Salze von Alkylsulfamidocarbonsäuren und die Salze von Alkyl- und Alkylarylethercarbonsäuren.

Des weiteren sind $C_8$-$C_{224}$-Olefinsulfonate, sulfonierte Polycarboxylsäuren, hergestellt durch Sulfonierung der Pyrrolyseprodukte von Erdalkalimetallcitraten, wie z. B. beschrieben in GB 1 082 179, Alkylglycerinsulfate, Fettacylglycerinsulfate, Oleylglycerinsulfate, Alkylphenolethersulfate, primäre Paraffinsulfonate, Alkylphosphate, Alkyletherphosphate, Isethionate, wie Acylisethionate, N-Acyltauride, Alkylsuccinamate, Sulfosuccinate, Monoester der Sulfosuccinate (besonders gesättigte und ungesättigte $C_{12}$-$C_{18}$-Monoester) und Diester der Sulfosuccinate (besonders gesättigte und ungesättigte $C_{12}$-$C_{18}$-Diester), Acylsarcosinate, Sulfate von Alkylpolysacchariden, wie Sulfate von Alkylglycosiden, verzweigte primäre Alkylsulfate und Alkylpolyethoxycarboxylate, wie die der Formel $RO(CH_2CH_2)_kCH_2COO\text{-}M+$, worin R ein $C_8$-$C_{22}$-Alkyl, k eine Zahl von 0 bis 10 und M ein lösliches Salz bildendes Kation ist, geeignet. Harzsäuren oder hydrierte Harzsäuren, wie Rosin oder hydriertes Rosin oder Tallölharze und Tallölharzsäuren sind einsetzbar. Weitere Beispiele sind in „Surface Active Agents and Detergents" (Vol. I und II, Schwartz, Perry und Berch) beschrieben. Eine Vielzahl solcher Tenside sind auch in US 3 929 678 beschrieben.

Typische Beispiele für anionische Tenside sind auch Alkylethersulfonate, Glycerinethersulfonate, Sulfofettsäuren, Fettalkoholethersulfate, Glycerinethersulfate, Hydroxymischethersulfat, Fettsäureamid-(ether)sulfate, Mono- und Dialkylsulfosuccinate, Mono- und Dialkylsulfosuccinamate, Sulfotriglyceride, Amidseifen, Alkyloligoglucosidsulfate, Alkylaminozuckersulfate und Alkyl-(ether)-phosphate. Sofern die anionischen Tenside Polyglycoletherketten enthalten, können sie eine konventionelle oder auch eingeengte Homologenverteilung aufweisen.

Des weiteren können in den erfindungsgemäßen melaninhaltigen Mitteln schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Als nichtionogene O/W-Emulgatoren kommen in Betracht Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; $C_{12}$-$C_{18}$-Fettsäuremono- und -diester von Anlagerungsprodukten von 1 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls geeignet sind Gemische von Verbindungen aus mehreren dieser Substanzklassen. Die Anlagerungsprodukte von Ethylenoxid und/oder Propylenoxid an Fettalkohole, Fettsäuren, Alkylphenole, Glycerinmono- und diester sowie Sorbitanmono- und - diester von Fettsäuren oder an Rizinusöl stellen bekannte, im Handel erhältliche Produkte dar. Es handelt sich dabei um Homologengemische, deren mittlerer Alkoxilierungsgrad dem Verhältnis der Stoffmengen von Ethylenoxid und/oder Propylenoxid und Substrat, mit denen die Anlagerungsreaktion durchgeführt wird, entspricht. $C_{12}$-$C_{18}$-Fettsäuremono- und -diester von Anlagerungaprodukten von Ethylenoxid an Glycerin sind aus DE-20 24 051 als Rückfettungsmittel für kosmetische Zubereitungen bekannt.

Des weiteren können eingesetzt werden Emulgatoren, erhalten durch Umesterung von gegebenenfalls oxalkyliertem Sorbit mit Fettsäuremethylestern oder Fettsäuretriglyceriden und gegebenenfalls Oxalkylierung der durch Umesterung mit Fettsäuremethylestern erhaltenen Reaktionsprodukte.

Als Überfettungsmittel können Substanzen, wie beispielsweise polyethoxilierte Lanolinderivate, Lecithinderivate, Polyolfettsäureester, Monoglyceride und Fettsäurealkanolamide verwendet werden, wobei die letzteren gleichzeitig als Schaumstabilisatoren dienen.

Typische Beispiele für Fette sind Glyceride, als Wachse kommen u. a. Bienenwachs, Paraffinwachs oder Mikrowachse, gegebenenfalls in Kombination mit hydrophilen Wachsen, z. B. Cetylstearylalkohol in Frage. Als Stabilisatoren können Metallsalze von Fettsäuren, wie z. B. Magnesium-, Aluminium- und/oder Zinkstearat eingesetzt werden.

Unter biogenen Wirkstoffen sind beispielsweise Pflanzenextrakte und Vitamine, beispielsweise Vitamin E und Vitamin C und Vitaminkomplexe sowie Antischuppenmittel, beispielsweise Octopyrox, zu verstehen. Als Konservierungsmittel eignen sich beispielsweise Phenoxyethanol, Formaldehydlösung, Parabene, Pentadiol oder Sorbinsäure.

Als Perlglanzmittel kommen beispielsweise Glycoldistearinsäureester, wie Ethylenglycoldistearat, aber auch Fettsäuremonoglycolester in Betracht.

Als Farbstoffe können die für kosmetische Zwecke geeigneten und zugelassenen Substanzen verwendet werden, wie sie beispielsweise in der Publikation „Kosmetische Färbemittel" der Farbstoffkommission der Deutschen Forschungsgemeinschaft, Verlag Chemie, Weinheim, 1984, S. 81 - 106 zusammengestellt sind.

Als Trägeröle kommen beispielsweise Guerbetalkohole mit 6 bis 18, vorzugsweise 8 bis 10 Kohlenstoffatomen, Ester von linearen $C_6$-$C_{13}$-Fettsäuren mit linearen $C_6$-$C_{20}$-Fettalkoholen, Ester von verzweigten $C_6$-$C_{13}$-Carbonsäuren mit linearen $C_6$-$C_{20}$-Fettalkoholen, Ester von linearen $C_6$-$C_{18}$-Fettsäuren mit verzweigten Alkoholen, insbesondere 2-Ethylhexanol, Ester von linearen und/oder verzweigten Fettsäuren mit mehrwertigen Alkoholen (wie z. B. Dimerdiol oder Trimerdiol) und/oder Guerbetalkoholen, Triglyceride auf Basis $C_6$-$C_{10}$-Fettsäuren, pflanzliche Öle, Mineralöle, Siliconöle, verzweigte primäre Alkohole, substituierte Cyclohexane, Guerbetcarbonate, Dialkylether und/oder aliphatische bzw. aromatische Kohlenwasserstoffe in Betracht.

Beispiel:

Der bei minus 80°C konservierte Pilz wurde aufgetaut und auf eine Agarplatte mit Biomalzmedium überimpft. Diese Platte wurde 12 Tage bei 30°C bebrütet, bis sich der Pilz braun gefärbt hatte. Im Anschluß wurde ein Agarstück mit Pilzmycel in physiologische Kochsatzlösung (Saline) überführt und mittels Glasperlen zerkleinert (Schütteln für 1 Min.). Die pilzhaltige Sahne wurde dann in die sterilisierte (121°C, 20 Min.) Vorkultur gegeben.

Vorkultur:

Volumen: 400 ml
Medium: Cornsteep 7,5 g/l
Malzextrakt 2,0 g/l

KH$_2$PO$_4$ 0,5 g/l
Glucose 100 g/l
Saccharose 5,0 g/l

| | |
|---|---|
| Gefäß: | 1 l-Erlenmeyerkolben |
| Schüttler: | 110 rpm |
| Temperatur: | 25°C |
| pH-Wert: | 6,5, ungeregelt |
| Antischaum: | 1ml Silikonentschäumer |
| Zeitdauer: | 4 Tage |

Die Vorkultur wurde im Anschluß steril in die Hauptkultur überführt. Die Hauptkultur wurde in einem 10 l-Fermenter durchgeführt.

Hauptkultur:

| | |
|---|---|
| Volumen: | 10 l |
| Medium: | Cornsteep 7,5 g/l |
| | Malzextrakt 2,0 g/l |
| | KH$_2$PO$_4$ 0,5 g/l |
| | Glucose 100 g/l |
| | Pharma Media 5,0 g/l |
| Drehzahl: | 250 rpm, Turbinenrührer |
| Begasung: | 0,2-0,9 vvm, Luft |
| pO2: | 70-90% |
| Temperatur: | 25°C |
| pH-Wert: | 6,5, geregelt mit 1 mol NaOH |
| Antischaum: | 10 ml Silikonentschäumer |
| Zeitdauer: | 7 Tage |

Zur Gewinnung der Melaninvorstufen wurde die Biomasse aus der Kulturlösung durch Zentrifugation bei 8.000 g abgetrennt, mit Wasser gewaschen und mit dem Turbinenrührer bei 900 mm aufgeschlossen. Mit einer Sörensenpufferlösung erfolgte die dreimalige Extraktion des Produktes und nach Einengung der Lösung mittels Rotationsverdampfer wurde das Produkt lyophilisiert. Die Ausbeute an Melanin betrug 10 g/l Medium der Hauptkultur.

**Patentansprüche**

1. Biomelanin, hergestellt durch eine Fermentation des Pilzes Podospora anserina der Familie Sondariaceae in einem geeigneten Nährmedium, enthaltend mindestens eine Kohlenstoff-, Phosphat- und Eiweißquelle bei einem pH von 5 - 7 und anschließende Aufarbeitung.

2. Biomelanin nach Anspruch 1, hergestellt durch Fermentation bei 15 bis 50 °C.

3. Biomelanin nach Anspruch 1, hergestellt durch eine Fermentationszeit von 4 bis 8 Tagen.

4. Kosmetische Zubereitungen enthaltend ein Biomelanin gemäß Anspruch 1.